# EUROPEAN PATENT APPLICATION

(11) **EP 4 591 919 A1**
(43) Date of publication of application: **30.07.2025**
(21) Application number: 23899681.3
(22) Date of filing: 07.11.2023
(51) Int. Cl.: A61M 60/174

(54) **BLOOD PUMP AND VENTRICULAR ASSISTANCE SYSTEM**

(30) Priority: 09.12.2022 CN 202211580636
(71) Applicant: Shenzhen Core Medical Technology Co., Ltd., Shenzhen, Guangdong 518000 (CN)
(72) Inventor: YU, Shunzhou, Shenzhen, Guangdong 518000 (CN); XIAO, Zhenzhong, Shenzhen, Guangdong 518000 (CN); YANG, Yuzhuo, Shenzhen, Guangdong 518000 (CN)
(74) Representative: Appleyard Lees IP LLP
(86) International application number: PCT/CN2023/130152
(87) International publication number: WO 2024/120100

(57) **Abstract**

A blood pump (10) and a ventricular assistance system. The blood pump (10) comprises a pump housing (11), a pipeline assembly (13), and an impeller (15). The pump housing (11) is provided with an accommodating cavity (112). The pipeline assembly (13) comprises a liquid outflow pipe (133) in communication with the accommodating cavity (112). The liquid outflow pipe (133) is provided with a liquid outlet (1332). The pipeline assembly (13) is further provided with a liquid inlet (1312). The liquid inlet (1312) is in communication with the accommodating cavity (112). The pipeline assembly (13) can penetrate a ventricular wall, such that the liquid inlet (1312) is located in a ventricle. The liquid outflow pipe (133) has such a length that when the pipeline assembly (13) penetrates the ventricular wall, the liquid outflow pipe (133) can extend through the ventricle and penetrate an aortic valve, so that the liquid outlet (1332) is located in an aorta. The impeller (15) can be rotatably accommodated in the accommodating cavity (112).

## Description

### CROSS-REFERENCE TO RELATED APPLICATIONS

This application claims priority to Chinese Patent Application No. 202211580636.9, filed on December 9, 2022, the entire content of which is incorporated herein by reference in its entirety.

### TECHNICAL FIELD

The present application relates to the technical field of medical devices, and in particular to a blood pump and a ventricular assistance system.

### BACKGROUND

Blood pump is a device connected between a ventricle and a blood vessel to assist a patient with severe ventricular insufficiency or heart failure to provide a certain blood flow and blood pressure. The conventional blood pump is connected to the human aorta by providing an artificial blood vessel outside the heart, and a hole needs to be made in the aorta to transport the blood in the ventricle to the aorta through the blood pump and the artificial blood vessel. However, making a hole on the aorta can easily lead to aortic diseases such as dissection and hematoma, and the operation is difficult and needs to be improved.

### SUMMARY

Based on this, the present application provides a blood pump and ventricular assistance system that avoids aortic diseases caused by making a hole on an aorta and reduces the difficulty of operation.

An embodiment of a first aspect of the present application provides a blood pump, which includes:
a pump housing provided with an accommodating cavity;
a pipeline assembly including a liquid outlet pipe in communication with the accommodating cavity, wherein the liquid outlet pipe is provided with a liquid outlet, the pipeline assembly is further provided with a liquid inlet in communication with the accommodating cavity, the pipeline assembly is capable of extending through a ventricular wall, so as to enable the liquid inlet to be located in a ventricle, the liquid outlet pipe has a certain length such that when the pipeline assembly extends through the ventricular wall, the liquid outlet pipe is capable of extending through the ventricle to extend through an aortic valve, so as to locate the liquid outlet in the aorta; and
an impeller, wherein the impeller is rotatably received in the accommodating cavity, so as to enable liquid entering the accommodating cavity from the liquid inlet to flow out from the liquid outlet through the liquid outlet pipe.

An embodiment of a second aspect of the present application provides a ventricular assistance system including a blood pump, and the blood pump includes:
a pump housing provided with an accommodating cavity;
a pipeline assembly including a liquid outlet pipe in communication with the accommodating cavity, wherein the liquid outlet pipe is provided with a liquid outlet, the pipeline assembly is further provided with a liquid inlet in communication with the accommodating cavity, the pipeline assembly is capable of extending through a ventricular wall, so as to enable the liquid inlet to be located in a ventricle, the liquid outlet pipe has a certain length such that when the pipeline assembly extends through the ventricular wall, the liquid outlet pipe is capable of extending through the ventricle to extend through an aortic valve, so as to locate the liquid outlet in the aorta; and
an impeller, wherein the impeller is rotatably received in the accommodating cavity, so as to enable the liquid entering the accommodating cavity from the liquid inlet to flow out from the liquid outlet through the liquid outlet pipe.

The details of one or more embodiments of the application are set forth in the accompanying drawings and the description below. Other features, objects, and advantages of the application will be apparent from the description and drawings, and from the claims.

### BRIEF DESCRIPTION OF THE DRAWINGS

In order to illustrate the embodiments of the present application more clearly, the drawings used in the embodiments or the conventional art will be described briefly. Apparently, the following described drawings are merely for the embodiments of the present application, and other drawings can be derived by those of ordinary skill in the art without any creative effort.
FIG. 1 is a schematic structural view of a blood pump according to a first embodiment of the present application.
FIG. 2 is a top view of the blood pump shown in FIG. 1.
FIG. 3 is a cross-sectional view taken along line A-A of FIG. 2.
FIG. 4 is a cross-sectional view taken along line B-B of FIG. 2.
FIG. 5 is an exploded view of the blood pump shown in FIG. 1 viewed from an aspect.
FIG. 6 is an exploded view of the blood pump shown in FIG. 1 viewed from another aspect.
FIG. 7 is a side view of the blood pump shown in FIG. 1.
FIG. 8 is a cross-sectional view taken along line C-C of FIG. 7.
FIG. 9 is an exploded view of a bottom housing and an impeller of the blood pump shown in FIG. 1.
FIG. 10 is an assembly view of the bottom housing and the impeller of the blood pump shown in FIG. 1.
FIG. 11 is a cross-sectional view taken along line D-D of FIG. 10.
FIG. 12 is a schematic structural view of the blood pump shown in FIG. 1 after being assembled with a driving motor.
FIG. 13 is a cross-sectional view taken along line E-E of FIG. 7.
FIG. 14 is a schematic structural view of a blood pump according to a second embodiment of the present application.
FIG. 15 is a cross-sectional view taken along line F-F of FIG. 14.
FIG. 16 is a partial exploded view of the blood pump shown in FIG. 14.

### DETAILED DESCRIPTION OF THE EMBODIMENTS

The present application will now be described in detail with reference to the accompanying drawings and embodiments in order to make the objects, technical solutions, and advantages of the present application clearer. It should be understood that the specific embodiments described herein are only for explaining the present application, and not intended to limit the present application.

It should be noted that when an element is referred to as being "fixed to" or "provided on" another element, it can be directly on the other element or an intervening element may also be present. When an element is referred to as being "connected to" another element, it can be directly connected to the other element or intervening elements may also be present.

In addition, the terms "first" and "second" are only used for descriptive purposes and cannot be understood as indicating or implying relative importance or implicitly indicating the number of indicated technical features. Thus, the features defined with "first" and "second" may explicitly or implicitly include one or more of the features. In the description of the present application, "a plurality of' means two or more, unless otherwise expressly and specifically defined.

In order to illustrate the technical solution of the present application, the following will be described in conjunction with specific drawings and embodiments.

As used herein, a proximal end is defined as an end adjacent to a medical staff, and a distal end is defined as an end away from the medical staff.

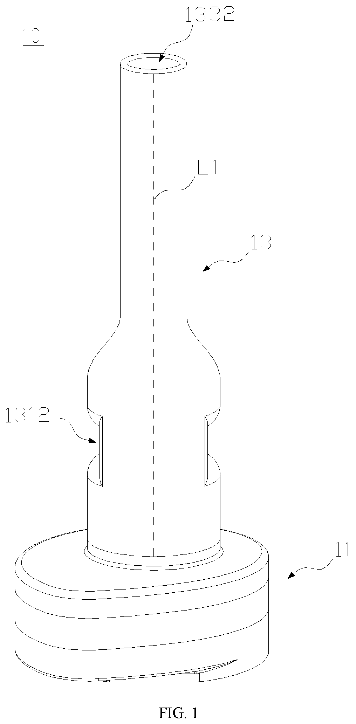

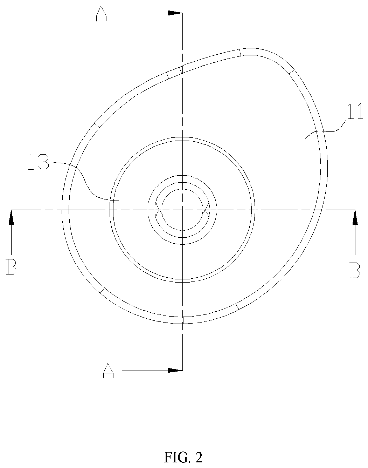

Referring to FIGS. 1 to 3, the present application provides a blood pump 10, including a pump housing 11, a pipeline assembly 13, and an impeller 15. The pump housing 11 is provided with an accommodating cavity 112. The pipeline assembly 13 has a first liquid flow channel 136 and a second liquid flow channel 138 both in communication with the accommodating cavity 112. The first liquid flow channel 136 has a first opening away from the accommodating cavity 112. The second liquid flow channel 138 has a second opening away from the accommodating cavity 112. The pipeline assembly 13 can extend into a first organ. When the pipeline assembly 13 extends into the first organ, the first opening is located in the first organ and is in communication with the first organ. The pipeline assembly 13 has a certain length (specifically, the length herein refers to a length of the pipeline assembly 13 in an extending direction thereof) such that when the pipeline assembly 13 extends into the first organ, the pipeline assembly 13 can extend through an interior of the first organ to a second organ in communication with the first organ, and the second opening is in communication with the second organ. The impeller 15 is rotatably received in the accommodating cavity 112 to enable liquid entering the accommodating cavity 112 from one of the first opening and the second opening to flow out through the other of the first opening and the second opening.

When the pipeline assembly 13 of the blood pump 10 provided by the present application extends into the first organ, the first opening is located in the first organ and is in communication with the first organ. The pipeline assembly 13 has a certain length L1, such that the pipeline assembly 13 extends through the interior of the first organ into the second organ in communication with the first organ, and the second opening is in communication with the second organ. Under the action of the rotation of the impeller 15, the liquid enters the accommodating cavity 112 through one of the first opening and the second opening, and flows out through the other of the first opening and the second opening. The liquid in the first organ can be pumped into the second organ, or the liquid in the second organ can be pumped into the first organ without an external artificial pipeline. That is, by adopting the above-mentioned the blood pump 10, there is no need to make a hole on the second organ, so that diseases caused by making a hole on the second organ can be avoided while satisfying the auxiliary pumping of blood. In addition, since the blood pump 10 does not require an external artificial pipeline, there is no need to sort out a layout path of the artificial pipeline, thereby reducing the difficulty of the operation.

Referring to FIGS. 1 to 3, a first embodiment of the present application is described by taking a left ventricle of a heart as the first organ and an aorta as the second organ.

The blood pump 10 according to the first embodiment of the present application includes a pump housing 11, a pipeline assembly 13, and an impeller 15. The pump housing 11 is provided with an accommodating cavity 112. The pipeline assembly 13 includes a liquid outlet pipe 133 in communication with the accommodating cavity 112, and the liquid outlet pipe 133 is provided with a liquid outlet 1332. Specifically, the liquid outlet 1332 is a second opening for the liquid to flow out, and the second liquid flow channel 138 is an outflow channel for the liquid. The pipeline assembly 13 further is provided with a liquid inlet 1312 in communication with the accommodating cavity 112. Specifically, the liquid inlet 1312 is a first opening for the inflow of liquid. The first liquid flow channel 136 is an inflow channel for the liquid. The pipeline assembly 13 can extend through a ventricular wall. When the pipeline assembly 13 extends through the ventricular wall, the liquid inlet 1312 is located in the ventricle and is in communication with the ventricle. The liquid outlet pipe 133 has a certain length L1, such that when the pipeline assembly 13 extends through the ventricular wall, the liquid outlet pipe 133 can extend through the ventricle to extend through the aortic valve, so as to enable the liquid outlet 1332 to be located in the aorta, and to enable the liquid outlet 1332 to be in communication with the aorta. The impeller 15 is rotatably received in the accommodating cavity 112 to enable the liquid entering the accommodating cavity 112 from the liquid inlet 1312 to flow out from the liquid outlet 1332 through the liquid outlet pipe 133.

The length L1 refers to a length of the liquid outlet pipe 133 in an extending direction thereof.

The liquid outlet pipe 133 of the blood pump 10 provided by the present application has a certain length L1, such that when the blood pump 10 is mounted in the left ventricle, the blood pump 10 is mounted in the left ventricle, the pump housing 11 is located outside the heart, the pipeline assembly 13 extends through the ventricular wall, the liquid inlet 1312 is located in the left ventricle, and the liquid outlet pipe 133 extends through the aortic valve through the ventricle to enable the liquid outlet 1332 to be located in the aorta. Under the action of the rotation of the impeller 15, the blood in the ventricle is sucked into the accommodating cavity 112 from the liquid inlet 1312 and flows to the aorta through the liquid outlet 1332, so that the blood in the ventricle is pumped into the aorta, and the blood in the left ventricle can be pumped into the aorta without an external artificial blood vessel. That is, by adopting the above-mentioned the blood pump 10, there is no need to make a hole on the aorta, so that aortic diseases such as dissection, hematoma and the like caused by the operation of making a hole on the aorta are avoided while satisfying the auxiliary pumping of blood. In addition, since the blood pump 10 does not require an external artificial pipeline, there is no need to sort out a layout path of the artificial blood vessel, the difficulty of the operation is reduced, and the blood leakage caused by the connection of the artificial blood vessel is eliminated. In addition, since the heart and other organs are closely attached to each other in the thoracic cavity, the blood pump 10 provided by the present application can extend the pipeline assembly 13 directly from the ventricle to the aorta without connecting any external artificial blood vessel, which greatly reduces the space occupied in the thoracic cavity and avoids other diseases caused by the contact between the artificial blood vessel and other organs.

The accommodating cavity 112 has a first cavity surface 1121 and a second cavity surface 1122 opposite to the first cavity surface 1121, and the impeller 15 is provided between the first cavity surface 1121 and the second cavity surface 1122. That is, the first cavity surface 1121 and the second cavity surface 1122 are provided on opposite sides of the impeller 15, and face two surfaces of the impeller 15, respectively.

In this embodiment, the pump housing 11 is further provided with a liquid outlet cavity 114, and the liquid outlet cavity 114 communicates the liquid outlet pipe 133 with the accommodating cavity 112. The liquid entering the accommodating cavity 112 from the liquid inlet 1312 can flow into the liquid outlet pipe 133 through the liquid outlet cavity 114. Specifically, the accommodating cavity 112 and the liquid outlet cavity 114 are provided along an axial direction of the impeller 15. The liquid outlet cavity 114 is located closer to the first cavity surface 1121 than the second cavity surface 1122.

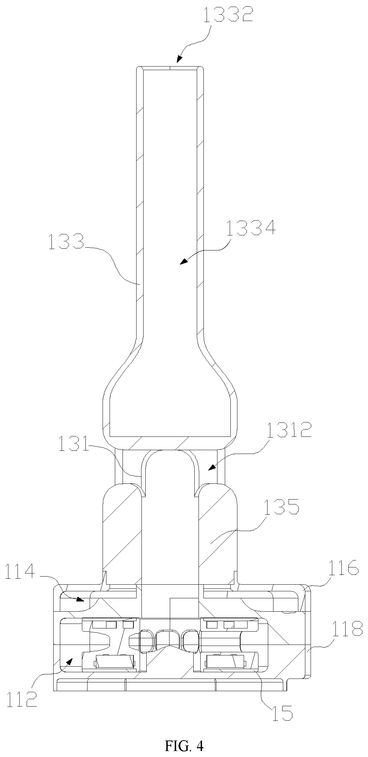

Referring to FIGS. 4 to 6, the pump housing 11 includes a bottom housing 118, and the accommodating cavity 112 is provided in the bottom housing 118. The pump housing 11 further includes a top housing 116, which is connected to the bottom housing 118 to cooperatively enclose the liquid outlet cavity 114. The top housing 116 is connected to the liquid outlet pipe 133.

The top housing 116 includes a main body 1162 and a flange 1164. The main body 1162 covers the bottom housing 118, and an outer peripheral surface of the main body 1162 is coplanar with an outer peripheral surface of the bottom housing 118. The main body 1162 is provided with an outflow through hole 1166 in communication with the liquid outlet cavity 114. The flange 1164 protrudes from the main body 1162 and surrounds the outflow through hole 1166. The flange 1164 is in the shape of an annular wall, and the flange 1164 is coaxial with the outflow through hole 116. The flange 1164 is connected to the liquid outlet pipe 133, and the outflow through hole 1166 is in communication with the liquid outlet pipe 133. The liquid flowing out of the liquid outlet cavity 114 flows to the liquid outlet pipe 133 through the outflow through hole 1166, and finally flows out from the liquid outlet 1332.

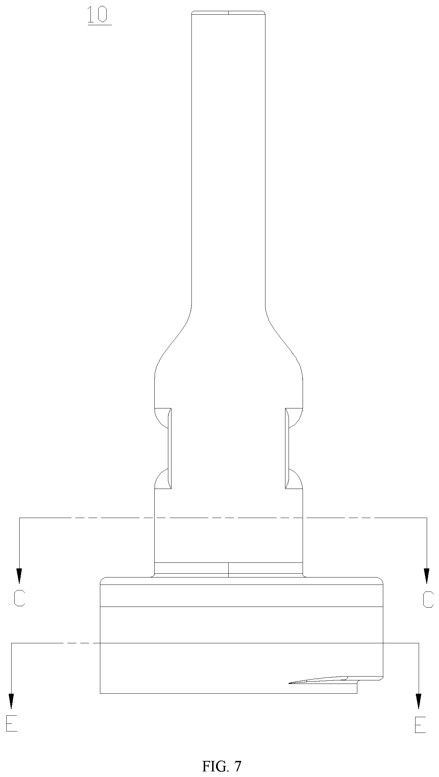

Referring to FIGS. 7 to 9, the bottom housing 118 is substantially in the shape of a volute. The bottom housing 118 includes a first housing wall 1181 and a second housing wall 1182 opposite to the first housing wall 1181. The accommodating cavity 112 is located between the first housing wall 1181 and the second housing wall 1182. The first cavity surface 1121 is provided on the first housing wall 1181, and the second cavity surface 1122 is provided on the second housing wall 1182. That is, the impeller 15 is located between the first housing wall 1181 and the second housing wall 1182. The first housing wall 1181 is connected to the top housing 116, and the first housing wall 1181 and the top housing 116 together enclose the liquid outlet cavity 114.

The bottom housing 118 further has a peripheral wall connected to the first housing wall 1181 and the second housing wall 1182, so as to enclose the accommodating cavity 112 together with the first housing wall 1181 and the second housing wall 1182. The peripheral wall has an inner peripheral surface 1184.

In one embodiment, the inner peripheral surface 1184 is an involute surface. The involute surface refers to a surface that extends in the manner of an involute. In a top view direction (i.e., in the axial direction of the impeller 15), the inner peripheral surface 1184 is substantially in an involute shape in a top view.

A flow guiding protrusion 1186 is provided in the liquid outlet cavity 114, and the position of the flow guiding protrusion 1186 corresponds to the position of an opening of an end of the liquid outlet pipe 133 away from the liquid outlet 1332, so as to guide the liquid in the liquid outlet cavity 114 to the liquid outlet pipe 133 and flow out through the liquid outlet port 1332. Specifically, the flow guiding protrusion 1186 protrudes from a housing wall of the bottom housing 118. More specifically, the flow guiding protrusion 1186 protrudes from the first housing wall 1181. A flow guiding surface 1185 is provided on an outer periphery of the flow guiding protrusion 1186, and the flow guiding surface 1185 is a conical surface to guide out the liquid in the liquid outlet cavity 114. The flow guiding surface 1185 may be the entire outer peripheral surface of the flow guiding protrusion 1186, or may be a portion of the outer peripheral surface of the flow guiding protrusion 1186. In this embodiment, the position of the flow guiding protrusion 1186 is substantially corresponding to the position of the impeller 15, so that the positions where the liquid flows in and out through the pipeline assembly 13 can be relatively concentrated. Since the liquid inlet 1312 and the liquid outlet 1332 are both provided on the pipeline assembly 13, the position of the flow guiding protrusion 1186 substantially corresponding to the position of the impeller 15 can facilitate the miniaturization of the pipeline assembly 13, thereby facilitating the mounting of the pipeline assembly 13 to the aortic valve. In this embodiment, the flow guiding protrusion 1186 is substantially a disc-shaped structure. In other embodiments, the flow guiding protrusion 1186 may be in the shape of a circular truncated cone or a double truncated cone, etc., as long as it is able to guide the liquid out of the liquid outlet cavity 114.

The bottom housing 118 further includes a joint pipe portion 1187 provided in the liquid outlet cavity 114 and in communication with the first liquid flow channel 136. Specifically, the joint pipe portion 1187 protrudes from the flow guiding protrusion 1186, and the position of the joint pipe portion 1187 corresponds to the position of the opening of the end of the liquid outlet pipe 133 away from the liquid outlet 1332. The liquid in the liquid outlet cavity 114 can flow to the liquid outlet 1332 through the flow guiding protrusion 1186 and the joint pipe portion 1187 in sequence. More specifically, the joint pipe portion 1187 protrudes from a middle portion of the flow guiding protrusion 1186. In this embodiment, the joint pipe portion 1187 is substantially a hollow cylindrical structure. In other embodiments, the joint pipe portion 1187 may have another structure such as a substantially hollow cuboid, which can be specifically set according to actual conditions.

The bottom housing 118 is further provided with a liquid flow through hole 1188 in communication with the joint pipe portion 1187. The liquid flow through hole 1188 extends through the housing wall of the bottom housing 118 and the flow guiding protrusion 1186. In this embodiment, the liquid flow through hole 1188 extends through the middle portion of the flow guiding protrusion 1186, and the liquid flow through hole 1188 may be a circular hole. In other embodiments, the liquid flow through hole 1188 can extends through other positions of the flow guiding protrusion 1186, and the liquid flow through hole 1188 may also be a square hole, a triangular hole, an elliptical hole, or other types of holes, which can be specifically set according to actual conditions.

The bottom housing 118 further includes a first housing 1189 and a second housing 1180. The first housing 1189 cooperates with the second housing 1180 to form the accommodating cavity 112. The first housing 1189 is connected to the top housing 116. By providing the first housing 1189 and the second housing 1180, the impeller 15 can be easily mounted into the accommodating cavity 112.

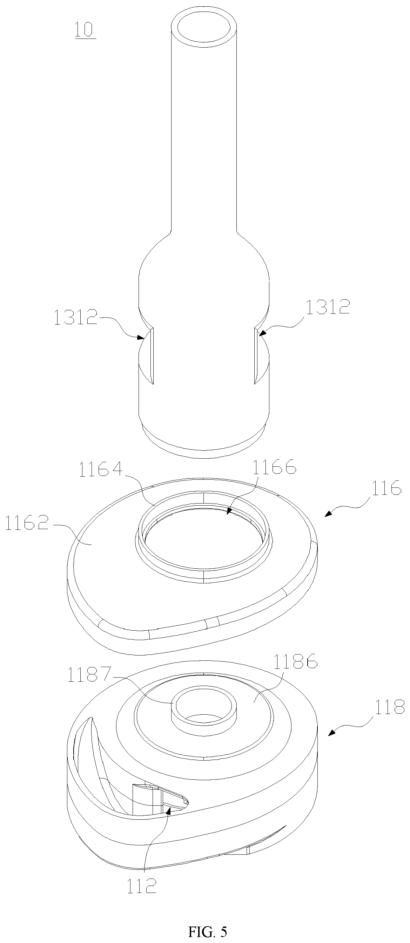

Referring to FIG. 5, the pump housing 11 is further provided with a communication flow channel 119 communicating the accommodating cavity 112 and the liquid outlet cavity 114. The liquid in the accommodating cavity 112 can flow into the liquid outlet cavity 114 through the communication flow channel 119. Specifically, the communication flow channel 119 is provided in the bottom housing 118. The communication flow channel 119 has a connecting surface connecting the second cavity surface 1122 and the liquid outlet cavity 114, and at least a part of the connecting surface is a flow guiding inclined surface 1191. The flow guiding inclined surface 1191 is configured to guide the liquid in the accommodating cavity 112 to the liquid outlet cavity 114. The flow guiding inclined surface 1191 can guide the liquid to flow from the accommodating cavity 112 into the liquid outlet cavity 114. In this embodiment, a distance between the flow guiding inclined surface 1191 and the second cavity surface 1122 gradually increases from an end of the flow guiding inclined surface 1191 adjacent to the accommodating cavity 112 to an end of the flow guiding inclined surface 1191 away from the accommodating cavity 112. Specifically, the flow guiding inclined surface 1191 connects a surface of the second housing wall 1182 facing the first housing wall 1181 and a surface of the first housing wall 1181 facing the liquid outlet cavity 114.

Specifically, the second cavity surface 1122 is provided on a side of the second housing wall 1182 facing the first housing wall 1181. The second cavity surface 1122 may be a surface of the entire second housing wall 1182 facing the first housing wall 1181, or the second cavity surface 1122 may be a portion of the surface of the second housing wall 1182 facing the first housing wall 1181. The surface of the first housing wall 1181 facing the liquid outlet cavity 114 is a surface of the first housing wall 1181 away from the first cavity surface 1121.

It should be noted that flow guiding inclined surface 1191 may be the entire connecting surface, or the connecting surface may be a portion of the connecting surface connecting the second cavity surface 1122 and the liquid outlet cavity 114. For example, in some embodiments, the flow guiding inclined surface 1191 is a section of the connecting surface adjacent to the liquid outlet cavity 114. In some embodiments, the flow guiding inclined surface 1191 is a section of the connecting surface adjacent to the accommodating cavity 112. In some embodiments, the flow guiding inclined surface 1191 is a middle section of the connecting surface in an extending direction of the communication flow channel 119.

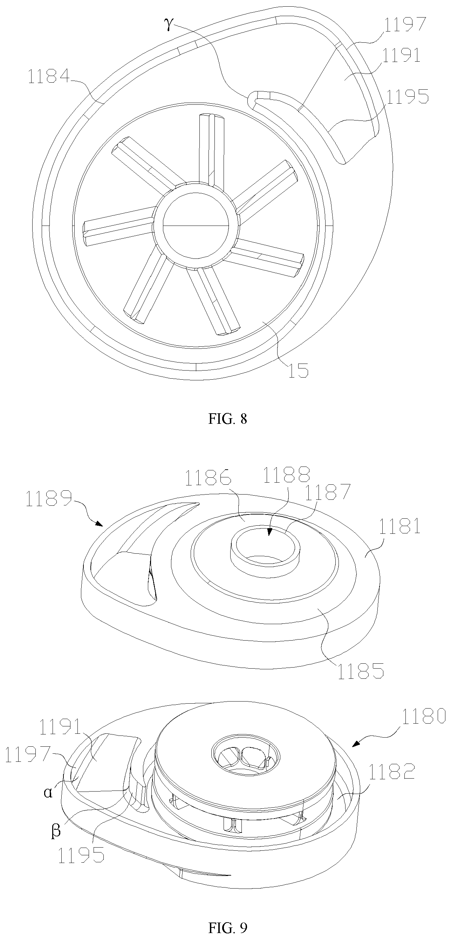

Referring to FIGS. 9 to 11, an angle θ between the flow guiding inclined surface 1191 and the surface of the second housing wall 1182 facing the first housing wall 1181 is 135° to 155°. In this embodiment, along the flow direction of the liquid, θ decreases first and then increases, and is always between 135° and 155°, so that the smoothness of the liquid flow can be improved. In other embodiments, along the flow direction of the liquid, θ is a fixed value, and the value of θ is between 135° and 155°. In this way, the blood pump 10 can be miniaturized and the burden on the heart can be reduced while improving the stability of the liquid flowing from the accommodating cavity 112 to the liquid outlet cavity 114. Under the condition that the distance between the surface of the second housing wall 1182 facing the first housing wall 1181 and the first cavity surface 1121 remains unchanged, when θ is greater than 155°, a length of the flow guiding inclined surface 1191 needs to be increased so that the flow guiding inclined surface 1191 can be connected to the surface of the second housing wall 1182 facing the first housing wall 1181 and the first cavity surface 1121, which will increase the volume of the bottom housing 118 and thus increase the volume of the blood pump 10. When θ is less than 135°, the flow speed of the liquid toward the liquid outlet cavity 114 will be slowed down, which is not conducive to the smooth flow of the liquid.

In this embodiment, a width of the communication flow channel 119 gradually decreases along the flow direction of the liquid. In the illustrated embodiment, the flow direction of the liquid refers to a direction in which the liquid flows from the accommodating cavity 112 to the outlet cavity 114 through the connecting channel 119. The width of the communication flow channel 119 gradually decreases along the flow direction of the liquid, so that the liquid is gradually tightened along the flow direction, thereby accelerating the flow speed of the liquid toward the liquid outlet cavity 114, weakening the adverse effect caused by the gravity factor of the liquid, and increasing the flow rate and speed of pumping the blood. In one embodiment, the width of the communication flow channel 119 is reduced in a linear manner. In another embodiment, the width of the communication flow channel 119 is reduced in a non-linear manner, for example, in an exponential manner.

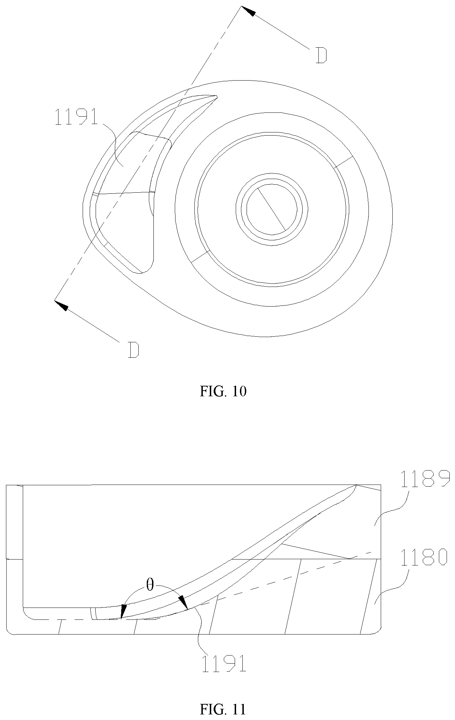

In this embodiment, as shown in FIG. 11, the communication flow channel 119 extends along a second-order or higher Bezier curve, which improves the smoothness of the liquid flow, thereby achieving a more stable pumping blood flow field. In one embodiment, the communication flow channel 119 may also extend along an arc line. In another embodiment, the communication flow channel 119 may also extend along an oblique line, as long as it can accelerate the flow speed of the liquid toward the liquid outlet cavity 114 and increase the flow rate and speed of pumping the blood.

In this embodiment, the width of the communication flow channel 119 gradually decreases along the flow direction of the liquid, and the angle θ between the flow guiding inclined surface 1191 and the surface of the second housing wall 1182 facing the first housing wall 1181 is 135° to 155°. In one embodiment, the width of the communication flow channel 119 gradually decreases along the flow direction of the liquid, and the angle θ between the flow guiding inclined surface 1191 and the surface of the second housing wall 1182 facing the first housing wall 1181 is not limited. In another embodiment, the width of the communication flow channel 119 along the flow direction of the liquid is not limited, and the angle θ between the flow guiding inclined surface 1191 and the surface of the second housing wall 1182 facing the first housing wall 1181 is 135° to 155°.

The communication flow channel 119 further has a first side surface 1195 and a second side surface 1197 that are both connected to the flow guiding inclined surface 1191. A connection between the first side surface 1195 and the flow guiding inclined surface 1191 has a rounded corner α, and a connection between the second side surface 1197 and the flow guiding inclined surface 1191 has a rounded corner β (as shown in FIG. 9). By providing the rounded corners, the sharp edges of the connection can be reduced and the hemolysis phenomenon can be reduced. Radiuses of the rounded corners α and β can be 0.1 mm to 0.5 mm. When the radiuses of the rounded corners α and β are less than 0.1 mm, the improvement of the hemolysis phenomenon is not obvious. When the radiuses of the rounded corners α and β are greater than 0.5 mm, the outflow of the blood will be hindered, which may easily cause the flow rate of pumping the blood to be too small. In other embodiments, the connection between the first side surface 1195 and the flow guiding inclined surface 1191, and the connection between the second side surface 1197 and the flow guiding inclined surface 1191 may not be provided with rounded corners, which can be specifically set according to actual conditions.

The first side surface 1195 is connected to one end of the inner peripheral surface 1184, and the second side surface 1197 is connected to the other end of the inner peripheral surface 1184. A rounded corner γ (as shown in FIG. 8) is formed at a connection between the first side surface 1195 and the inner peripheral surface 1184. A radius of the rounded corner γ is 0.2 mm to 0.5 mm. If the radius of the rounded corner γ is less than 0.2 mm, a sharp corner will be formed at the connection between the inner peripheral surface 1184 and the first side surface 1195, which easily leads to hemolysis. If the radius of the rounded corner γ is greater than 0.5 mm, the outflow of blood will be hindered, thereby reducing the flow rate of the pumping the blood.

In this embodiment, the second side surface 1197 is also an involute surface, that is, a top view of the second side surface 1197 is substantially involute-shaped. In other embodiments, the second side surface 1197 may also be an arc surface.

Continuing to refer to FIGS. 1, 4 and 5, the pipeline assembly 13 is connected to the pump housing 11. The liquid inlet 1312 is closer to the proximal end of the pipeline assembly 13 than the liquid outlet 1332, and the liquid inlet 1312 is configured for the inflow liquid (e.g., blood). The liquid outlet 1332 is provided at the proximal end of the pipeline assembly 13 and is configured for the outflow of the liquid. The liquid outlet 1332 is in communication with the liquid outlet cavity 114, so that the pipeline assembly 13 has the functions of liquid inlet and liquid outlet at the same time, that is, the liquid can enter the pump housing 11 through the pipeline assembly 13 and can flow out through the pipeline assembly 13.

The liquid outlet pipe 133 is connected to the pump housing 11 and is in communication with the accommodating cavity 112. For example, the liquid outlet pipe 133 is connected to the flange 1164 of the top housing 116, and the liquid in the liquid outlet cavity 114 can also flow to the liquid outlet pipe 133 along the inner peripheral surface of the flange 1164 and the inner peripheral surface of the liquid outlet pipe 133 and flow out from the liquid outlet 1332. In this embodiment, the inner peripheral surface of the liquid outlet pipe 133 may be coplanar with the inner peripheral surface of the flange 1164, so that the liquid flows out of the liquid outlet cavity 114 more stably and smoothly. The liquid inlet 1312 is exposed on the outer peripheral surface of the liquid outlet pipe 133, and the liquid outlet 1332 is provided on the liquid outlet pipe 133.

The liquid outlet pipe 133 can extend through the ventricular wall and extend from the pump housing 11 to extend through the aortic valve, so that the liquid outlet 1332 is located in the aorta, and the blood in the accommodating cavity 112 can enter the aorta through the liquid outlet 1332, so as to convey the blood in the ventricle from the interior of the heart to the aorta.

The liquid outlet pipe 133 has a liquid outlet tube cavity 1334, and the liquid outlet tube cavity 1334 is in communication with the liquid outlet 1332 and the accommodating cavity 112. Specifically, the liquid outlet cavity 114 is in communication with the liquid outlet 1332 through the liquid outlet tube cavity 1334.

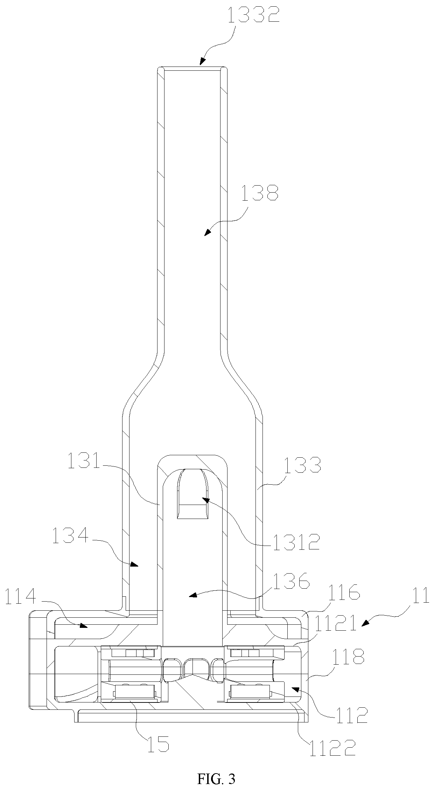

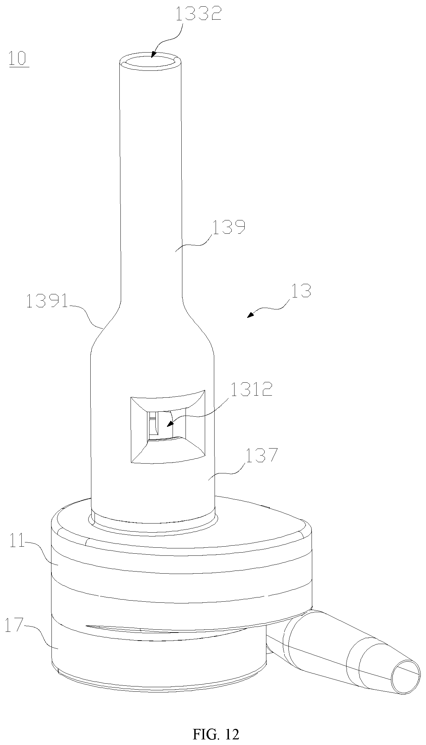

Referring to FIG. 3 and FIG. 12, in this embodiment, the pipeline assembly 13 includes a first pipe section 137 and a second pipe section 139 that are both provided in the liquid outlet pipe 133. The first pipe section 137 is connected between the second pipe section 139 and the pump housing 11. The liquid inlet 1312 is provided in the first pipe section 137, and the liquid outlet 1332 is provided in the second pipe section 139. In this embodiment, an outer diameter of the first pipe section 137 is greater than an outer diameter of the second pipe section 139, that is, an outer diameter of the distal end of the liquid outlet pipe 133 is less than an outer diameter of the proximal end of the pipeline assembly 13, which can reduce the damage of the liquid outlet pipe 133 to the aorta. In this embodiment, the second pipe section 139 is provided with a transition section 1391 connected to the first pipe section 137. Along a direction from the first pipe section 137 to the second pipe section 139, an outer diameter of the transition section 1391 gradually decreases, so as to avoid sharp edges and facilitate the safe insertion of the pipeline assembly 13 into the ventricle. In this embodiment, a generatrix of the transition section 1391 may be an arc line or a second-order Bezier curve. In other embodiments, the generatrix of the transition section 1391 may also be an oblique line.

In this embodiment, the pipeline assembly 13 further includes a liquid inlet pipe 131 in communicated with the liquid inlet pipe 131. The liquid inlet pipe 131 is at least partially provided in the liquid outlet pipe 133, and a length of the liquid inlet pipe 131 is less than that of the liquid outlet pipe 133. Specifically, at least part of the liquid inlet pipe 131 is provided in the first pipe section 137. In one embodiment, the liquid inlet pipe 131 may be entirely provided in the liquid outlet pipe 133. In another embodiment, part of the liquid inlet pipe 131 is located in the liquid outlet pipe 133, and the other part extends out from the liquid outlet pipe 133.

A liquid flow channel 134 is formed between the liquid inlet pipe 131 and the liquid outlet pipe 133. The liquid flow channel 134 is at least partially located between the liquid inlet pipe 131 and the first pipe section 137. In this embodiment, the inner peripheral surface of the liquid inlet pipe 131 encloses the first liquid flow channel 136. The liquid flow channel 134 is the second liquid flow channel 138. The liquid flow channel 134 is in communication with both the liquid outlet 1332 and the accommodating cavity 112. The liquid can flow into the liquid inlet pipe 131 from the liquid inlet 1312, and then flow out from the liquid outlet 1332 through the accommodating cavity 112 and the liquid flow channel 134 in sequence.

Referring to FIG. 3, FIG. 6 and FIG. 9, the liquid inlet pipe 131 is connected to the pump housing 11. Specifically, the liquid inlet pipe 131 is connected to the first housing wall 1181 of the bottom housing 118, the liquid inlet pipe 131 is in communication with the accommodating cavity 112, and the liquid inlet 1312 is in communication with the liquid inlet pipe 131. In this embodiment, the liquid inlet pipe 131 is connected to the joint pipe portion 1187 in the liquid outlet cavity 114, thereby separating the first liquid flow channel 136 from the second liquid flow channel 138, preventing the liquid flow in the first liquid flow channel 136 and the liquid flow in the second liquid flow channel 138 from influencing each other, and improving the smoothness and stability of the liquid inflow and outflow, thus increasing the flow rate and speed of pumping the blood. The liquid inlet pipe 131 is connected to the joint pipe portion 1187 in the liquid outlet cavity 114 and is in communication with the liquid flow through hole 1188, so that the liquid in the liquid outlet cavity 114 can flow to the liquid outlet pipe 133 along the outer peripheral surface of the joint pipe portion 1187 and the outer peripheral surface of the liquid inlet pipe 131, and flow out from the liquid outlet 1332. In this embodiment, the outer peripheral surface of the liquid inlet pipe 131 may be coplanar with the outer peripheral surface of the joint pipe portion 1187, so that the liquid flows out from the liquid outlet cavity 114 more stably and smoothly. In other embodiments, the bottom housing 118 may not include the joint pipe portion 1187, and the end of the liquid inlet pipe 131 adjacent to the pump housing 11 may protrude from the liquid outlet pipe 133 and be connected to the flow guiding protrusion 1186, or the liquid inlet pipe 131 is not connected to the flow guiding protrusion 1186, but directly extends into the accommodating cavity 112, both of which can allow the liquid to enter the accommodating cavity 112 through the liquid inlet pipe 131.

The liquid inlet pipe 131 is in communication with the liquid flow through hole 1188, and the liquid flow through hole 1188 extends through the middle portion of the flow guiding protrusion 1186, so that the positions where the liquid flows in and out through the pipeline assembly 13 can be relatively concentrated, thereby facilitating the miniaturization of the pipeline assembly 13.

In this embodiment, the liquid inlet 1312 is in communication with the liquid inlet pipe 131, the liquid inlet 1312 is not in communication with the liquid outlet pipe cavity 1334, and the liquid outlet pipe cavity 1334 is in communication with the liquid outlet 1332. Therefore, the liquid entering the liquid inlet pipe 131 from the liquid inlet 1312 and the liquid flowing out from the liquid outlet pipe cavity 1334 to the liquid outlet 1332 will not affect each other, thereby improving the smoothness and stability of liquid inflow and outflow, thus increasing the flow rate and speed of pumping the blood.

In this embodiment, a plurality of liquid inlets 1312 are provided, the plurality of liquid inlets 1312 are arranged along a circumferential direction perpendicular to an axis of the liquid outlet pipe 133 and are exposed on the outer peripheral surface of the liquid outlet pipe 133. The plurality of liquid inlets 1312 are all in communication with the liquid inlet pipe 131, so that the liquid entering through any one of the plurality of liquid inlets 1312 can flow into the accommodating cavity 112. When a certain liquid inlet 1312 is blocked by the ventricular wall when the blood enters, the blood can still enter the ventricle from other liquid inlets 1312 to achieve normal inflow and outflow of the blood.

In this embodiment, two liquid inlets 1312 are provided that are opposite to each other, so that when the liquid enters the blood pump 10 from the two liquid inlets 1312, the force on the blood pump 10 is relatively balanced, thereby avoiding tilting caused by unbalanced force. In one embodiment, the positions of the two liquid inlets 1312 may not correspond to each other. In another embodiment, only one liquid inlet 1312 may be provided, which may be set according to actual conditions.

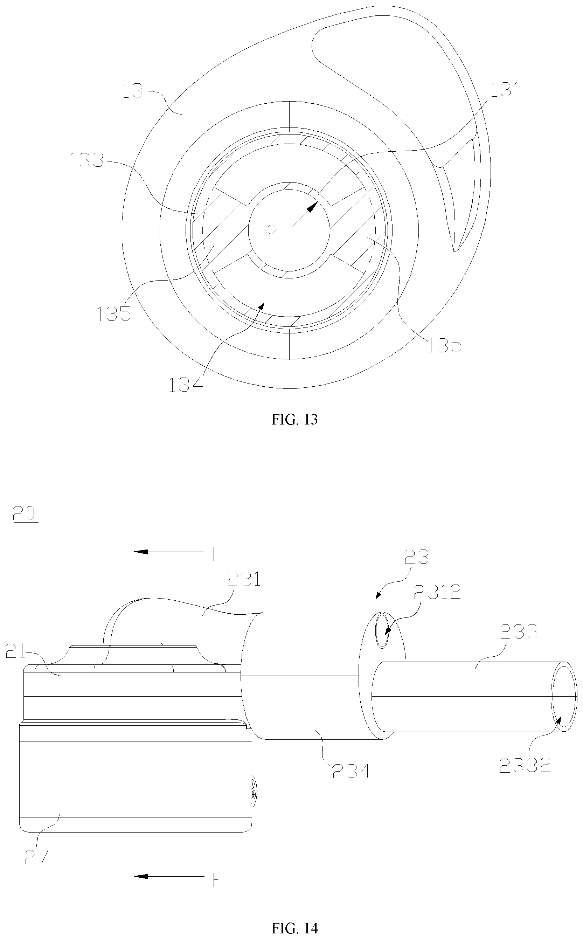

Referring to FIG. 13, in this embodiment, an inner diameter of the end of the liquid inlet pipe 131 adjacent to the accommodating cavity 112 is d, and a total cross-sectional area of the end of the liquid flow channel 134 adjacent to the accommodating cavity 112 is S, and S=πd²/4. That is, the sum of the areas of the two sector rings shown in FIG. 13 is equal to an area of an inner cross-sectional of the liquid inlet pipe 131. In this way, a cross-sectional area of a liquid inflow area is equal to a cross-sectional area of a liquid outflow area, so that the flow rate of the liquid entering the blood pump 10 is substantially equal to the flow rate of the liquid flowing out of the blood pump 10, thereby improving the stability of the flow rate of the pumping the blood.

The pipeline assembly 13 further includes a connecting member 135 connected between the liquid inlet pipe 131 and the liquid outlet pipe 133, and the connecting member 135 is configured to fix the liquid inlet pipe 131 inside the liquid outlet pipe 133. In this embodiment, two connecting members 135 are provided along a circumferential direction of the liquid inlet pipe 131 and are located at both radial ends of the liquid inlet pipe 131. In other embodiments, one, three, or more connecting members 135 may be provided, as long as the purpose of fixing the liquid inlet pipe 131 inside the liquid outlet pipe 133 is achieved.

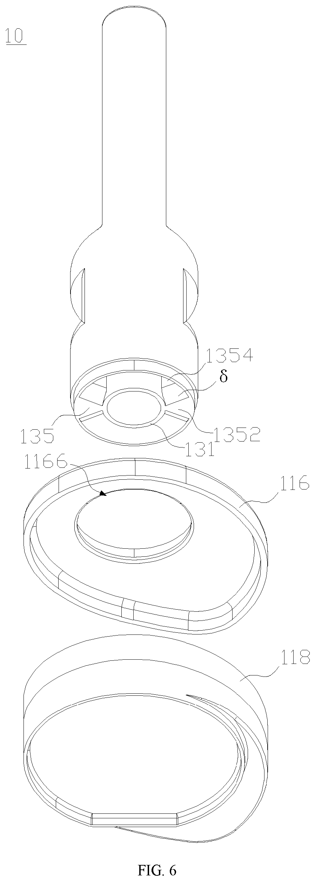

Referring to FIG. 6, the connecting member 135 includes a connecting bottom surface 1352 and a connecting side surface 1354 connected to each other. The connecting bottom surface 1352 faces the liquid outlet cavity 114, so that the liquid flowing out of the liquid outlet cavity 114 can be in contact with the connecting bottom surface 1352. The connecting side surface 1354 is opposite to the inner wall of the liquid outlet pipe 133, and the liquid flowing out of the liquid outlet cavity 114 can also be in contact with the connecting side surface 1354. In this embodiment, a connection between the connecting bottom surface 1352 and the connecting side surface 1354 has a rounded corner δ (as shown in FIG. 6) to reduce hemolysis. As an example, a radius of the rounded corner δ may be 0.1 mm to 0.5 mm. In other embodiments, the radius of the rounded corner δ may also be other values, which is not limited herein.

It should be noted that, in other embodiments, the pipeline assembly 13 may not include the liquid inlet pipe 131, that is, the pipeline assembly 13 only includes the liquid outlet pipe 133. The liquid outlet pipe 133 may include the first liquid flow channel 136 and the second liquid flow channel 13, the first liquid flow channel 136 and the second liquid flow channel 138 are separated by a partition plate, and the partition plate is connected to the inner wall of the liquid outlet pipe 133. The first liquid flow channel 136 is in communication with the liquid inlet 1312, and the second liquid flow channel 138 is in communication with the liquid outlet 1332, which can also achieve that when the impeller 15 is operated, the liquid entering the accommodating cavity 112 from the liquid inlet 1312 can flow out from the liquid outlet 1332 through the liquid outlet pipe 133.

The impeller 15 is rotatably received in the accommodating cavity 112 to convey the liquid entering the accommodating cavity 112 from the liquid inlet 1312 to the liquid outlet 1332 through the liquid outlet cavity 114. A rotor (not shown) may be provided in the impeller 15. The rotor may cooperate with a stator of the driving motor. When the stator drives the rotor to rotate in the accommodating cavity 112, the impeller 15 also rotates synchronously with the rotor in the accommodating cavity 112. The specific structure of the impeller 15 may refer to the prior art.

The blood pump 10 further includes a driving motor 17 connected to a side of the pump housing 11 away from the pipeline assembly 13. The driving motor 17 cooperates with the impeller 15 to drive the impeller 15 to rotate. For example, an interior of the driving motor 17 includes a stator that magnetically cooperates with the rotor in the impeller 15 to cause the rotor to rotate, thereby driving the impeller 15 to rotate.

A second embodiment of the present application is described by taking the left ventricle of the heart as the first organ and the aorta as the second organ.

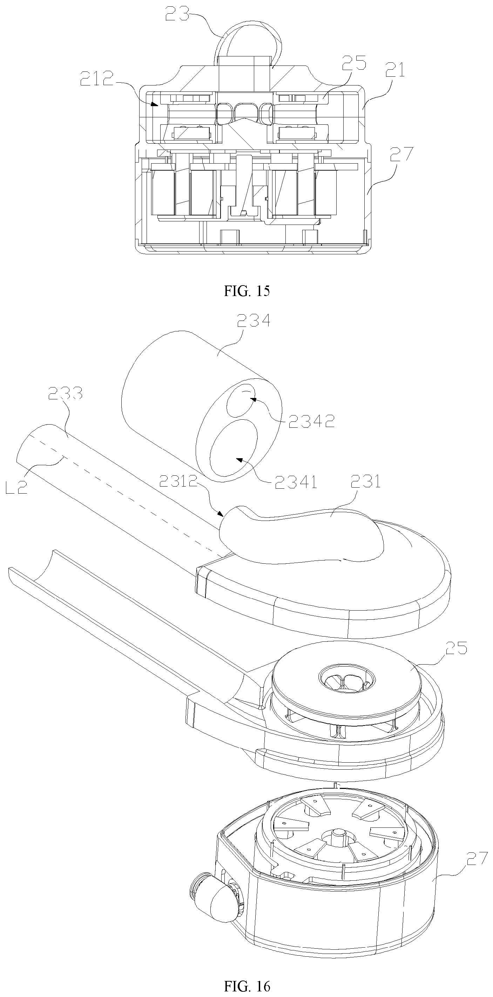

Referring to FIGS. 14 to 16, a blood pump 20 according to the second embodiment of the present application includes a pump housing 21, a pipeline assembly 23, and an impeller 25. The pump housing 21 is provided with an accommodating cavity 212. The pipeline assembly 23 includes a liquid outlet pipe 233 in communication with the accommodating cavity 212. The liquid outlet pipe 233 is connected to the pump housing 21 and is provided with a liquid outlet 2332. Specifically, the liquid outlet 2332 is a second opening for the outflow of liquid. The pipeline assembly 23 further has a liquid inlet 2312 in communication with the accommodating cavity 212. Specifically, the liquid inlet 2312 is a first opening for the inflow of liquid. The pipeline assembly 23 can extend through the ventricular wall. When the pipeline assembly 23 extends through the ventricular wall, the liquid inlet 2312 is located in the ventricle and is in communication with the ventricle. The liquid outlet pipe 233 has a certain length L2 such that when the pipeline assembly 23 extends through the ventricular wall, the liquid outlet pipe 233 can extend through the ventricle to extend through the aortic valve, so as to enable the liquid outlet 2332 to be located in the aorta, and to enable the liquid outlet 2332 to be in communication with the aorta. The impeller 25 is rotatably received in the accommodating cavity 212 to allow the liquid entering the accommodating cavity 212 from the liquid inlet 2312 to flow out through the liquid outlet 2332.

As different from the blood pump 10, the pump housing 21 of the blood pump 20 includes an outer peripheral surface that is at least partially arc-shaped, and the liquid outlet pipe 233 extends from the pump housing 21 along a tangential direction of the arc-shaped outer peripheral surface, so that the blood pump 20 can flow out along the tangential direction of the arc-shaped outer peripheral surface of the pump housing 21 under the action of the rotation of the impeller 25. In addition, the blood pump 20 does not include the top housing 116 (see FIG. 5). The liquid outlet pipe 233 is connected to the bottom housing 218, and the bottom housing 218 does not include the flow guiding inclined surface 1191 (see FIG. 13). The blood entering the accommodating cavity 212 through the liquid inlet 2312 is directly conveyed to the liquid outlet 2332 of the liquid outlet pipe 233 under the rotation of the impeller 25, and flows to the aorta without passing through other structures such as the liquid outlet cavity 114 (see FIG. 3). Therefore, the delivery of blood is smoother and the flow rate of pumping the blood is increased.

The blood pump 20 further includes a liquid inlet pipe 231 connected to the pump housing 21 and provided outside the liquid outlet pipe 233. The liquid inlet pipe 231 is in communication with the accommodating cavity 212, and the liquid inlet 2312 is in communication with the liquid inlet pipe 231. The liquid inlet pipe 231 is bent and extends from the pump housing 21 in a direction approaching the liquid outlet pipe 233, so that the liquid outlet pipe 233 extends through the ventricular wall while the liquid inlet pipe 231 can also extend through the ventricular wall, and the liquid inlet 2312 is located in the ventricle. The blood in the ventricle can be pumped to the aorta without additionally making a hole on the heart to place the liquid inlet pipe 231 and without providing an artificial blood vessel, thereby avoiding making a hole on the aorta.

The liquid inlet 2312 is provided on the liquid inlet pipe 231 and is in communication with the accommodating cavity 212 through a cavity of the liquid inlet pipe 231. The liquid outlet pipe 233 is provided outside the liquid inlet pipe 231, and the liquid outlet 2332 is in communication with the accommodating cavity 212 through a cavity of the liquid outlet pipe 233. That is, the liquid inlet pipe 231 and the liquid outlet pipe 233 of the blood pump 20 are independent from each other, and the cavity of the liquid inlet pipe 231 and the cavity of the liquid outlet pipe 233 are not communicated with each other. An inner peripheral wall of the liquid inlet pipe 231 encloses a first liquid flow channel, and an inner peripheral wall of the liquid outlet pipe 233 encloses a second liquid flow channel.

In this embodiment, the liquid inlet pipe 231 extends from the pump housing 21 toward the liquid outlet pipe 233, so that the liquid inlet pipe 231 and the liquid outlet pipe 233 face the same direction, which facilitates the subsequent fixed connection of the liquid inlet pipe 231 and the liquid outlet pipe 233, so that the liquid inlet pipe 231 can extend through the ventricular wall while the liquid outlet pipe 233 extends through the aortic valve.

The pipeline assembly 23 further includes a connecting sleeve 234, which can extend through the ventricular wall. The connecting sleeve 23 is provided with a first connecting hole 2341 and a second connecting hole 2342 that are spaced apart. The liquid outlet pipe 233 extends through the first connecting hole 2341. For example, the liquid outlet pipe 233 extends through the first connecting hole 2341 and extends in a direction away from the pump housing 21. The liquid inlet pipe 231 is connected to the connecting sleeve 234, and the liquid inlet pipe 231 is in communication with the second connecting hole 2342. The liquid inlet 2312 is an opening of the second connecting hole 2342, that is, the liquid inlet 2312 may be provided on the connecting sleeve 234. Alternatively, the liquid inlet pipe 231 extends through the second connecting hole 2342, and the liquid inlet 2312 is provided on the liquid inlet pipe 231. An end of the liquid inlet pipe 231 away from the pump housing 21 may be coplanar with an end surface of the connecting sleeve 234 or protrude from the end surface of the connecting sleeve 234. The connecting sleeve 234 is configured to fix the liquid inlet pipe 231 and the liquid outlet pipe 233. When mounting the blood pump the connecting sleeve 234 extends into the ventricular wall, both the liquid inlet pipe 231 and the liquid outlet pipe 233 can extends into the ventricular wall, and the liquid inlet pipe 231 does not need to extends into the heart wall additionally, which reduces the difficulty of the operation.

The blood pump 20 further includes a driving motor 27 connected to the pump housing 21, and the driving motor 27 cooperates with the impeller 25 to drive the impeller 25 to rotate.

In one embodiment, both the blood pump 10 and the blood pump 20 may be used in a right ventricle, that is, the right ventricle of the heart as the first organ and a pulmonary artery as the second organ. The first opening is the liquid inlet 1312 for the inflow of the liquid. The second opening is the liquid outlet 1332 for the outflow of the liquid. The first liquid flow channel 136 is surrounded by the inner peripheral surface of the liquid inlet pipe 131 (or the liquid inlet pipe 231). The second liquid flow channel 138 is surrounded by the outer peripheral surface of the liquid inlet pipe 131 and the inner peripheral surface of the liquid outlet pipe 133, or is only surrounded by the inner peripheral surface of the liquid outlet pipe 233. That is, when the blood pump 10 is mounted in the right ventricle of the heart, the pipeline assembly 13 can extend through the pulmonary valve, so that the first opening is located in the right ventricle and the second opening is located in the pulmonary artery. Therefore, the blood in the pulmonary artery can be pumped to the right ventricle without an external artificial blood vessel, thereby avoiding making a hole on the pulmonary artery. Pulmonary artery diseases caused by the operation of making a hole on the pulmonary artery are avoided while satisfying the auxiliary pumping of blood. In addition, there is no need to sort out a layout path of the artificial blood vessel, the difficulty of the operation is reduced, and the blood leakage caused by the clamping and connecting of the artificial blood vessel is eliminated.

In other embodiments, the blood pump 10 and the blood pump 20 may also be applied to other tissues similar to the heart, as long as the purpose of pumping liquid from the first organ to the second organ, or pumping liquid from the second organ to the first organ can be achieved.

In summary, according to the blood pump 10 and the blood pump 20 provided by the present application, the blood pump 10 includes the pump housing 11, the pipeline assembly 13, and the impeller 15. The pump housing 11 is provided with the accommodating cavity 112. The pipeline assembly 13 includes the liquid outlet pipe 133 in communication with the accommodating cavity 112. The liquid outlet pipe 133 is provided with the liquid outlet 1332. The pipeline assembly 13 is further provided with the liquid inlet 1312 in communication with the accommodating cavity 112. The pipeline assembly 13 can extend through the ventricular wall, so that the liquid inlet 1312 is located in the ventricle. The liquid outlet pipe 133 has the certain length L1, so that when the pipeline assembly 13 extends through the ventricular wall, the liquid outlet pipe 133 can extend through the ventricle to extend through the aortic valve, so as to enable the liquid outlet 1332 to be located in the aorta. The impeller 15 is rotatably received in the accommodating cavity 112 to allow the liquid entering the accommodating cavity 112 from the liquid inlet 1312 to flow out from the liquid outlet 1332 through the liquid outlet pipe 133. The pipeline assembly 13 extends through the ventricle wall to enable the liquid inlet 1312 to be located in the ventricle, and the liquid outlet pipe 133 has the certain length L1, when the pipeline assembly 13 extends through the ventricle wall, the liquid outlet pipe 133 extend through the ventricle to extend through the aortic valve, so that the liquid outlet 1332 is located in the aorta, and the blood in the ventricle can be pumped into the aorta without an external artificial blood vessel. That is, by adopting the above-mentioned the blood pump 10, there is no need to make a hole on the aorta, so that aortic diseases such as dissection, hematoma and the like caused by the operation of making a hole on the aorta are avoided while satisfying the auxiliary pumping of blood. In addition, since the blood pump 10 does not require an external artificial pipeline, there is no need to sort out a layout path of the artificial blood vessel, the difficulty of the operation is reduced, and the problem of blood leakage caused by the connection of the artificial blood vessel is eliminated. In addition, since the heart and other organs are closely attached to each other in the thoracic cavity, the blood pump 10 provided by the present application can extend the pipeline assembly 13 directly from the ventricle to the aorta without connecting any external artificial blood vessel, which greatly reduces the space occupied in the thoracic cavity and avoids other diseases caused by the contact between the artificial blood vessel and other organs.

It should be noted that the blood pump 10 and the blood pump 20 can also be used for the right ventricle, and the blood in the pulmonary artery can be pumped to the right ventricle without an external artificial blood vessel, thereby avoiding making a hole on the pulmonary artery. Pulmonary artery diseases caused by the operation of making a hole on the pulmonary artery are avoided while satisfying the auxiliary pumping of blood, and there is no need to sort out a layout path of the artificial blood vessel, the difficulty of the operation is reduced, and the blood leakage caused by the clamping and connecting of the artificial blood vessel is eliminated. In addition, the blood pump 10 may also be applied to other tissues similar to the heart, as long as the purpose of pumping liquid from the first organ to the second organ, or pumping liquid from the second organ to the first organ can be achieved.

The present application further provides a ventricular assistance system (not shown), including the blood pump 10 (or the blood pump 20). The ventricular assistance system may further include a ventricular connection assembly (not shown) and a console (not shown). The ventricular connection assembly may be configured to mount the blood pump 10 to the heart. The console may be electrically connected to the blood pump 10 to control the operation of the blood pump 10. Structures or devices such as the ventricular connection assembly and the console may refer to the prior art, and are not specifically limited herein.

The ventricular assistance system provided by the present application includes the blood pump 10. Since the blood pump 10 can avoid aortic diseases such as dissection, hematoma and the like caused by the operation of making a hole on the aorta, the difficulty of the operation is reduced, and other diseases caused by the contact of the artificial blood vessel and other organs are avoided. Therefore, according to the ventricular assistance system provided by the present application, the difficulty of the operation can also be reduced, and other diseases caused by the contact of the artificial blood vessel and other organs can also be avoided.

The above embodiments are only used to illustrate the technical solutions of the present application, rather than to limit it. Although the present application has been described in detail with reference to the foregoing embodiments, those skilled in the art should understand that the technical solutions described in the foregoing embodiments can still be modified, or some of the technical features thereof can be equivalently replaced. These modifications or replacements made on the basis of not violating the concept of the technical solutions of the embodiments of the present application are within the protection scope of the present application.

## Claims

1. A blood pump, comprising:
a pump housing provided with an accommodating cavity;
a pipeline assembly comprising a liquid outlet pipe in communication with the accommodating cavity, wherein the liquid outlet pipe is provided with a liquid outlet, the pipeline assembly is further provided with a liquid inlet in communication with the accommodating cavity, the pipeline assembly is capable of extending through a ventricular wall, so as to enable the liquid inlet to be located in a ventricle, the liquid outlet pipe has a certain length such that when the pipeline assembly extends through the ventricular wall, the liquid outlet pipe is capable of extending through the ventricle to extend through an aortic valve, so as to locate the liquid outlet in the aorta; and
an impeller, wherein the impeller is rotatably received in the accommodating cavity, so as to enable liquid entering the accommodating cavity from the liquid inlet to flow out from the liquid outlet through the liquid outlet pipe.

2. The blood pump according to claim 1, wherein the pipeline assembly further comprises a liquid inlet pipe, the liquid inlet pipe is at least partially provided in the liquid outlet pipe and is in communication with the accommodating cavity, a liquid flow channel is formed between the liquid inlet pipe and the liquid outlet pipe, the liquid flow channel communicates the liquid outlet with the accommodating cavity, the liquid inlet is exposed on an outer peripheral surface of the liquid outlet pipe, and the liquid inlet is in communication with the liquid inlet pipe.

3. The blood pump according to claim 2, wherein the pipeline assembly further comprises a connecting member connected between the liquid inlet pipe and the liquid outlet pipe, the connecting member comprises a connecting bottom surface and a connecting side surface that are connected to each other, the connecting bottom surface faces the accommodating cavity, the connecting side surface is connected to an inner surface of the liquid outlet pipe, and a connection between the connecting bottom surface and the connecting side surface has a rounded corner.

4. The blood pump according to claim 2, wherein an inner diameter of an end of the liquid inlet pipe adjacent to the accommodating cavity is d, a total cross-sectional area of the end of the liquid flow channel adjacent to the accommodating cavity is S, and S=πd²/4.

5. The blood pump according to claim 2, wherein a plurality of liquid inlets are provided, the plurality of liquid inlets are arranged along a circumferential direction perpendicular to an axis of the liquid outlet pipe and are exposed from the outer peripheral surface of the liquid outlet pipe, and the plurality of liquid inlets are in communication with the liquid inlet pipe.

6. The blood pump according to claim 5, wherein two liquid inlets are provided that are opposite to each other.

7. The blood pump according to claim 2, wherein the pipeline assembly comprises a first pipe section and a second pipe section, the first pipe section is connected between the second pipe section and the pump housing, the liquid inlet is provided on the first pipe section, the liquid outlet is provided on the second pipe section, and an outer diameter of the first pipe section is greater than an outer diameter of the second pipe section.

8. The blood pump according to claim 1, wherein the pump housing is further provided with a liquid outlet cavity, the liquid outlet cavity communicates the liquid outlet pipe with the accommodating cavity, and the liquid entering the accommodating cavity from the liquid inlet flows into the liquid outlet pipe through the liquid outlet cavity.

9. The blood pump according to claim 8, wherein the accommodating cavity has a first cavity surface and a second cavity surface opposite to the first cavity surface, the impeller is located between the first cavity surface and the second cavity surface, the liquid outlet cavity is adjacent to the first cavity surface, the pump housing is further provided with a communication flow channel communicating the accommodating cavity with the liquid outlet cavity, the communication flow channel has a connecting surface connecting the second cavity surface and the liquid outlet cavity, and at least a part of the connecting surface is a flow guiding inclined surface.

10. The blood pump according to claim 8, wherein the pump housing comprises a bottom housing and a top housing, the accommodating cavity is provided in the bottom housing, the top housing is connected to the bottom housing to cooperatively enclose the liquid outlet cavity, and the liquid outlet pipe is connected to the top housing.

11. The blood pump according to claim 10, wherein the bottom housing comprises a first housing wall and a second housing wall opposite to the first housing wall, the accommodating cavity is located between the first housing wall and the second housing wall, the top housing and the first housing wall cooperatively enclose the liquid outlet cavity, the bottom housing is further provided with a communication flow channel communicating the accommodating cavity with the liquid outlet cavity, the communication flow channel has a flow guiding inclined surface, and the flow guiding inclined surface connects a surface of the second housing wall facing the first housing wall and a surface of the first housing wall facing the liquid outlet cavity.

12. The blood pump according to claim 9 or 11, wherein the communication flow channel further has a first side surface and a second side surface that are both connected to the flow guiding inclined surface, a connection between the first side surface and the flow guiding inclined surface has a rounded corner, and a connection between the second side surface and the flow guiding inclined surface has a rounded corner; and/or
a flow guiding protrusion is further provided in the liquid outlet cavity, and a position of the flow guiding protrusion corresponds to a position of an opening of an end of the liquid outlet pipe away from the liquid outlet.

13. The blood pump according to claim 11, wherein the communication flow channel further has a first side surface and a second side surface that are both connected to the flow guiding inclined surface, the bottom housing further has a peripheral wall, the peripheral wall is connected to the first housing wall and the second housing wall to cooperatively enclose the accommodating cavity, the peripheral wall has an inner peripheral surface, one end of the inner peripheral surface is connected to the first side surface, and another end of the inner peripheral surface is connected to the second side surface;wherein a rounded corner is formed at a connection between the inner peripheral surface and the first side surface, and a radius of the rounded corner is 0.2 mm to 0.5 mm;
and/or
the inner peripheral surface and the second side surface are both involute surfaces.

14. The blood pump according to claim 10, wherein the pipeline assembly further comprises a liquid inlet pipe in communication with the liquid inlet and the accommodating cavity, the bottom housing comprises a flow guiding protrusion and a joint pipe portion, the flow guiding protrusion protrudes from the housing wall of the bottom housing, the joint pipe portion protrudes from the flow guiding protrusion and is in communication with the liquid inlet pipe, the bottom housing is further provided with a liquid flow through hole in communication with the joint pipe portion, and the liquid flow through hole extends through the housing wall of the bottom housing and the flow guiding protrusion, so that the liquid inlet pipe is in communication with the accommodating cavity.

15. The blood pump according to claim 11, wherein a width of the communication flow channel gradually decreases along a flow direction of the liquid;
and/or an angle θ between the flow guiding inclined surface and the surface of the second housing wall facing the first housing wall is 135° to 155°.

16. The blood pump according to claim 1, wherein the pipeline assembly further comprises a liquid inlet pipe provided outside the liquid outlet pipe, the liquid inlet pipe is in communication with the accommodating cavity and the liquid inlet, and the liquid outlet pipe is provided outside the liquid inlet pipe.

17. The blood pump according to claim 16, wherein the liquid outlet pipe and the liquid inlet pipe are both connected to the pump housing, the pump housing comprises an outer peripheral surface that is at least partially arc-shaped, the liquid outlet pipe extends from the pump housing along a tangential direction of the arc-shaped outer peripheral surface, and the liquid inlet pipe is bent and extends from the pump housing in a direction approaching the liquid outlet pipe.

18. The blood pump according to claim 16, wherein the pipeline assembly further comprises a connecting sleeve capable of extending through the ventricular wall, the connecting sleeve is provided with a first connecting hole and a second connecting hole that are spaced apart, the liquid outlet pipe extends through the first connecting hole ;
wherein the liquid inlet pipe is connected to the connecting sleeve, the liquid inlet pipe is in communication with the second connecting hole, and the liquid inlet is an opening of the second connecting hole;
or, the liquid inlet pipe extends through the second connecting hole, and the liquid inlet is provided on the liquid inlet pipe.

19. A blood pump, comprising:
a pump housing provided with an accommodating cavity;
a pipeline assembly having a first liquid flow channel and a second liquid flow channel, wherein the first liquid flow channel and the second liquid flow channel are both in communication with the accommodating cavity, the first liquid flow channel has a first opening away from the accommodating cavity, the second liquid flow channel has a second opening away from the accommodating cavity, the pipeline assembly is capable of extending into a first organ, when the pipeline assembly extends into the first organ, the first opening is located in the first organ and is in communication with the first organ, the pipeline assembly has a certain length such that when the pipeline assembly extends into the first organ, the pipeline assembly is capable of extending through an interior of the first organ to a second organ in communication with the first organ, and the second opening is in communication with the second organ; and
an impeller rotatably received in the accommodating cavity to enable liquid entering the accommodating cavity from one of the first opening and the second opening to flow out through the other of the first opening and the second opening.

20. A ventricular assistance system, comprising a blood pump comprising:
a pump housing provided with an accommodating cavity;
a pipeline assembly comprising a liquid outlet pipe in communication with the accommodating cavity, wherein the liquid outlet pipe is provided with a liquid outlet, the pipeline assembly is further provided with a liquid inlet in communication with the accommodating cavity, the pipeline assembly is capable of extending through a ventricular wall, so as to enable the liquid inlet to be located in a ventricle, the liquid outlet pipe has a certain length such that when the pipeline assembly extends through the ventricular wall, the liquid outlet pipe is capable of extending through the ventricle to extend through an aortic valve, so as to locate the liquid outlet in the aorta; and
an impeller, wherein the impeller is rotatably received in the accommodating cavity, so as to enable the liquid entering the accommodating cavity from the liquid inlet to flow out from the liquid outlet through the liquid outlet pipe.
